# EUROPEAN PATENT SPECIFICATION

(11) **EP 1 141 278 B1**
(45) Date of publication and mention of the grant of the patent: **27.02.2008**
(21) Application number: 99968130.7
(22) Date of filing: 15.12.1999
(51) Int. Cl.: C12N 15/11, C07H 21/02, A61K 31/712, A61P 17/00, A61P 29/00

(54) **THERAPEUTIC PDE4D PHOSPHODIESTERASE INHIBITORS**
THERAPEUTISCHE PDE4D PHOSPHODIESTERASE INHIBITOREN
INHIBITEURS DE LA PHOSPHODIESTERASE PDE4D UTILISES A DES FINS THERAPEUTIQUES

(30) Priority: 30.12.1998 US 223586; 29.07.1999 US 364626
(43) Date of publication of application: 10.10.2001
(73) Proprietor: Oligos Etc. Inc., Wilsonville, OR 97070 (US)
(72) Inventor: DALE, Roderic, M., K., Wilsonville, OR 97070 (US); ARROW, Amy, Bethel, ME 04217 (US); THOMPSON, Terry, West Linn, OR 97068 (US)
(74) Representative: Viering, Jentschura & Partner
(86) International application number: PCT/US1999/029976
(87) International publication number: WO 2000/040714

(56) References cited:
- WO-A-94/15619
- WO-A-96/20281
- WO-A-98/13526
- HOUSLAY MD. ET AL.: "The multienzyme PDE4 cyclic adenosine monophosphate-specific phosphodiesterase family: intracellular targeting, regulation, and selective inhibition by compounds exerting anti-inflammatory and antidepressant actions." ADV PHARMACOL, vol. 44, 1998, pages 225-342, XP000906963
- ALTMANN, K.-H. ET AL.: "Novel chemistry" STEIN, C.A. & KRIEG, A.M. 'APPLIED ANTISENSE OLIGONUCLEOTIDE TECHNOLOGY'. WILEY-LISS, NEW YORK, US; CHAPTER 4, 1998, pages 73-107, XP002119324
- EPSTEIN PAUL M: "Antisense inhibition of phosphodiesterase expression." METHODS, vol. 14, no. 1, January 1998 (1998-01), pages 21-33, XP004466608 ISSN: 1046-2023

### SEQUENCE LISTING

<110> Dale, Roderic M. K.
   Arrow, Amy
   Thompson, Terry
<120> Antisense Phosphodiesterase Inhibitors
<130> OLIG-003WO
<150> 09/223,586
   <151> 1998-12-30
<150> 09/364,626
   <151> 1999-07-29
<160> 51
<170> FastSEQ for Windows Version 3.0
<210> 1
   <211> 25
   <212> DNA
   <213> Artificial Sequence
<220>
   <223> Synthesized Oligonucleotide
<400> 1
   ttagagcagg tctcgcagaa gaaat 25
<210> 2
   <211> 25
   <212> DNA
   <213> Artificial Sequence
<220>
   <223> Synthesized Oligonucleotide
<400> 2
   agcgtcagca tgtatgtcac catcg 25
<210> 3
   <211> 25
   <212> DNA
   <213> Artificial Sequence
<220>
   <223> Synthesized Oligonucleotide
<400> 3
   gcttgctgag gttctggaag atgtc 25
<210> 4
   <211> 24
   <212> DNA
   <213> Artificial Sequence
<220>
   <223> Synthesized Oligonucleotide
<400> 4
   agagcttcct cgactcctga caat 24
<210> 5
   <211> 25
   <212> DNA
   <213> Artificial Sequence
<220>
   <223> Synthesized Oligonucleotide
<400> 5
   atgttagagt tgttcaggct gttac 25
<210> 6
   <211> 25
   <212> DNA
   <213> Artificial Sequence
<220>
   <223> Synthesized Oligonucleotide
<400> 6
   aggagctctt cttgatcggt cttca 25
<210> 7
   <211> 23
   <212> DNA
   <213> Artificial Sequence
<220>
   <223> Synthesized Oligonucleotide
<400> 7
   gagaatctcc aggtccgtga aca 23
<210> 8
   <211> 18
   <212> DNA
   <213> Artificial Sequence
<220>
   <223> Synthesized Oligonucleotide
<400> 8
   ggcgctgtag taccagtc 18
<210> 9
   <211> 16
   <212> DNA
   <213> Artificial Sequence
<220>
   <223> Synthesized Oligonucleotide
<400> 9
   acagggacag aggtct 16
<210> 10
   <211> 25
   <212> DNA
   <213> Artificial Sequence
<220>
   <223> Synthesized Oligonucleotide
<400> 10
   gacttctagt cagtatcgcc aggag 25
<210> 11
   <211> 25
   <212> DNA
   <213> Artificial Sequence
<220>
   <223> Synthesized Oligonucleotide
<400> 11
   acaaatcaca gtggtgctct gcctg 25
<210> 12
   <211> 25
   <212> DNA
   <213> Artificial Sequence
<220>
   <223> Synthesized Oligonucleotide
<400> 12
   ggtcttctaa agtcatcatg taggt 25
<210> 13
   <211> 20
   <212> DNA
   <213> Artificial Sequence
<220>
   <223> Synthesized Oligonucleotide
<400> 13
   ctaaggtatc gagaatgtcc 20
<210> 14
   <211> 25
   <212> DNA
   <213> Artificial Sequence
<220>
   <223> Synthesized Oligonucleotide
<400> 14
   catgctcatc aaggatagaa tgttc 25
<210> 15
   <211> 23
   <212> DNA
   <213> Artificial Sequence
<220>
   <223> Synthesized Oligonucleotide
<400> 15
   gacgtttggg ttatataata cac 23
<210> 16
   <211> 25
   <212> DNA
   <213> Artificial Sequence
<220>
   <223> Synthesized Oligonucleotide
<400> 16
   ttgttagaag ccatctcact gacag 25
<210> 17
   <211> 25
   <212> DNA
   <213> Artificial Sequence
<220>
   <223> Synthesized Oligonucleotide
<400> 17
   atgtcaataa ccatcttcct gagtg 25
<210> 18
   <211> 25
   <212> DNA
   <213> Artificial Sequence
<220>
   <223> Synthesized Oligonucleotide
<400> 18
   tctaggagaa gaagccctga acttg 25
<210> 19
   <211> 20
   <212> DNA
   <213> Artificial Sequence
<220>
   <223> Synthesized Oligonucleotide
<400> 19
   gagttctatg cagactctca 20
<210> 20
   <211> 25
   <212> DNA
   <213> Artificial Sequence
<220>
   <223> Synthesized Oligonucleotide
<400> 20
   tcagtagttc gggagcattc agaag 25
<210> 21
   <211> 18
   <212> DNA
   <213> Artificial Sequence
<220>
   <223> Synthesized Oligonucleotide
<400> 21
   ttctccatgc gccagaga 18
<210> 22
   <211> 18
   <212> DNA
   <213> Artificial Sequence
<220>
   <223> Synthesized Oligonucleotide
<400> 22
   cagaggagtt ccgagaca 18
<210> 23
   <211> 18
   <212> DNA
   <213> Artificial Sequence
<220>
   <223> Synthesized Oligonucleotide
<400> 23
   cgaggcttgt caccttct 18
<210> 24
   <211> 25
   <212> DNA
   <213> Artificial Sequence
<220>
   <223> Synthesized Oligonucleotide
<400> 24
   tggccctaag tcctctggtt gtcga 25
<210> 25
   <211> 18
   <212> DNA
   <213> Artificial Sequence
<220>
   <223> Synthesized Oligonucleotide
<400> 25
   gcttggctgc tcctaggc 18
<210> 26
   <211> 23
   <212> DNA
   <213> Artificial Sequence
<220>
   <223> Synthesized Oligonucleotide
<400> 26
   atgtcctctc catgtaggtc gct 23
<210> 27
   <211> 20
   <212> DNA
   <213> Artificial Sequence
<220>
   <223> Synthesized Oligonucleotide
<400> 27
   gttcccactt acgtccgcca 20
<210> 28
   <211> 25
   <212> DNA
   <213> Artificial Sequence
<220>
   <223> Synthesized Oligonucleotide
<400> 28
   ccaagtctgt gaacacagcc tcgag 25
<210> 29
   <211> 25
   <212> DNA
   <213> Artificial Sequence
<220>
   <223> Synthesized Oligonucleotide
<400> 29
   cgattgtcct ccagcgtgtc cagca 25
<210> 30
   <211> 25
   <212> DNA
   <213> Artificial Sequence
<220>
   <223> Synthesized Oligonucleotide
<400> 30
   agtcatagct ctcttcagcc tccaa 25
<210> 31
   <211> 25
   <212> DNA
   <213> Artificial Sequence
<220>
   <223> Synthesized Oligonucleotide
<400> 31
   ttgcactgtt acgtgtcagg agaac 25
<210> 32
   <211> 14
   <212> DNA
   <213> Artificial Sequence
<220>
   <223> Synthesized Oligonucleotide
<400> 32
   cgtgtcagga gaac 14
<210> 33
   <211> 25
   <212> DNA
   <213> Artificial Sequence
<220>
   <223> Synthesized Oligonucleotide
<400> 33
   acaggcttca ggttggcttt cctct 25
<210> 34
   <211> 25
   <212> DNA
   <213> Artificial Sequence
<220>
   <223> Synthesized Oligonucleotide
<400> 34
   gaggctttgt tgggttgctc agatc 25
<210> 35
   <211> 25
   <212> DNA
   <213> Artificial Sequence
<220>
   <223> Synthesized Oligonucleotide
<400> 35
   agataatagc acatgagtag actgg 25
<210> 36
   <211> 25
   <212> DNA
   <213> Artificial Sequence
<220>
   <223> Synthesized Oligonucleotide
<400> 36
   catctcactg acggagtgcc tggtc 25
<210> 37
   <211> 18
   <212> DNA
   <213> Artificial Sequence
<220>
   <223> Synthesized Oligonucleotide
<400> 37
   taatggtctt cgagagtc 18
<210> 38
   <211> 25
   <212> DNA
   <213> Artificial Sequence
<220>
   <223> Synthesized Oligonucleotide
<400> 38
   ttgtacatca aggcaagttc agagt 25
<210> 39
   <211> 25
<212> DNA
   <213> Artificial Sequence
<220>
   <223> Synthesized Oligonucleotide
<400> 39
   gaagaactcc agagcttgtc acttt 25
<210> 40
   <211> 25
   <212> DNA
   <213> Artificial Sequence
<220>
   <223> Synthesized Oligonucleotide
<400> 40
   tgatcagaaa ttgattggac acacc 25
<210> 41
   <211> 25
   <212> DNA
   <213> Artificial Sequence
<220>
   <223> Synthesized Oligonucleotide
<400> 41
   tggtaccatt cacgattgtc ctcca 25
<210> 42
   <211> 25
   <212> DNA
   <213> Artificial Sequence
<220>
   <223> Synthesized Oligonucleotide
<400> 42
   tctcggagag atcactggag agagc 25
<210> 43
   <211> 25
   <212> DNA
   <213> Artificial Sequence
<220>
   <223> Synthesized Oligonucleotide
<400> 43
   atgtgccacc gtgaaacgcc gctgt 25
<210> 44
   <211> 18
   <212> DNA
   <213> Artificial Sequence
<220>
   <223> Synthesized Oligonucleotide
<400> 44
   tcctacgtta catgtagt 18
<210> 45
   <211> 20
   <212> DNA
   <213> Artificial Sequence
<220>
   <223> Synthesized Oligonucleotide
<400> 45
   catatccagg aatgccttct 20
<210> 46
   <211> 647
   <212> PRT
   <213> human
<400> 46
   Met Pro Leu Val Asp Phe Phe Cys Glu Thr Cys Ser Lys Pro Trp Leu
   1 5 10 15
   Val Gly Trp Trp Asp Gln Phe Lys Arg Met Leu Asn Arg Glu Leu Thr
   20 25 30
   His Leu Ser Glu Met Ser Arg Ser Gly Asn Gln Val Ser Glu Tyr Ile
   35 40 45
   Ser Thr Thr Phe Leu Asp Lys Gln Asn Glu Val Glu Ile Pro Ser Pro
   50 55 60
   Thr Met Lys Glu Arg Glu Lys Gln Gln Ala Pro Arg Pro Arg Pro Ser
   65 70 75 80
   Gln Pro Pro Pro Pro Pro Val Pro His Leu Gln Pro Met Ser Gln Ile
   85 90 95
   Thr Gly Leu Lys Lys Leu Met His Ser Asn Ser Leu Asn Asn Ser Asn
   100 105 110
   Ile Pro Arg Phe Gly Val Lys Thr Asp Gln Glu Glu Leu Leu Ala Gln
   115 120 125
   Glu Leu Glu Asn Leu Asn Lys Trp Gly Leu Asn Ile Phe Cys Val Ser
   130 135 140
   Asp Tyr Ala Gly Gly Arg Ser Leu Thr Cys Ile Met Tyr Met Ile Phe
   145 150 155 160
   Gln Glu Arg Asp Leu Leu Lys Lys Phe Arg Ile Pro Val Asp Thr Met
   165 170 175
   Val Thr Tyr Met Leu Thr Leu Glu Asp His Tyr His Ala Asp Val Ala
   180 185 190
   Tyr His Asn Ser Leu His Ala Ala Asp Val Leu Gln Ser Thr His Val
   195 200 205
   Leu Leu Ala Thr Pro Ala Leu Asp Ala Val Phe Thr Asp Leu Glu Ile
   210 215 220
   Leu Ala Ala Leu Phe Ala Ala Ala Ile His Asp Val Asp His Pro Gly
   225 230 235 240
   Val Ser Asn Gln Phe Leu Ile Asn Thr Asn Ser Glu Leu Ala Leu Met
   245 250 255
   Tyr Asn Asp Glu Ser Val Leu Glu Asn His His Leu Ala Val Gly Phe
   260 265 270
   Lys Leu Leu Gln Glu Asp Asn Cys Asp Ile Phe Gln Asn Leu Ser Lys
   275 280 285
   Arg Gln Arg Gln Ser Leu Arg Lys Met Val Ile Asp Met Val Leu Ala
   290 295 300
   Thr Asp Met Ser Lys His Met Thr Leu Leu Ala Asp Leu Lys Thr Met
   305 310 315 320
   Val Glu Thr Lys Lys Val Thr Ser Ser Gly Val Leu Leu Leu Asp Asn
   325 330 335
   Tyr Ser Asp Arg Ile Gln Val Leu Arg Asn Met Val His Cys Ala Asp
   340 345 350
   Leu Ser Asn Pro Thr Lys Pro Leu Glu Leu Tyr Arg Gln Trp Thr Asp
   355 360 365
   Arg Ile Met Ala Glu Phe Phe Gln Gln Gly Asp Arg Glu Arg Glu Arg
       370                 375                 380
   Gly Met Glu Ile Ser Pro Met Cys Asp Lys His Thr Ala Ser Val Glu
   385                 390                 395                 400
   Lys Ser Gln Val Gly Phe Ile Asp Tyr Ile Val His Pro Leu Trp Glu
                   405                 410                 415
   Thr Trp Ala Asp Leu Val His Pro Asp Ala Gln Glu Ile Leu Asp Thr
               420                 425                 430
   Leu Glu Asp Asn Arg Asp Trp Tyr Tyr Ser Ala Ile Arg Gln Ser Pro
           435                 440                 445
   Ser Pro Pro Pro Glu Glu Glu Ser Arg Gly Pro Gly His Pro Pro Leu
       450                 455                 460
   Pro Asp Lys Phe Gln Phe Glu Leu Thr Leu Glu Glu Glu Glu Glu Glu
   465                 470                 475                 480
   Glu Ile Ser Met Ala Gln Ile Pro Cys Thr Ala Gln Glu Ala Leu Thr
                   485                 490                 495
   Ala Gln Gly Leu Ser Gly Val Glu Glu Ala Leu Asp Ala Thr Ile Ala
               500                 505                 510
   Trp Glu Ala Ser Pro Ala Gln Glu Ser Leu Glu Val Met Ala Gln Glu
           515                 520                 525
   Ala Ser Leu Glu Ala Glu Leu Glu Ala Val Tyr Leu Thr Gln Gln Ala
       530                 535                 540
   Gln Ser Thr Gly Ser Ala Pro Val Ala Pro Asp Glu Phe Ser Ser Arg
   545                 550                 555                 560
   Glu Glu Phe Val Val Ala Val Ser His Ser Ser Pro Ser Ala Leu Ala
                   565                 570                 575
   Leu Gln Ser Pro Leu Leu Pro Ala Trp Arg Thr Leu Ser Val Ser Glu
               580                 585                 590
   His Ala Pro Gly Leu Pro Gly Leu Pro Ser Thr Ala Ala Glu Val Glu
           595                 600                 605
   Ala Gln Arg Glu His Gln Ala Ala Lys Arg Ala Cys Ser Ala Cys Ala
       610                 615                 620
   Gly Thr Phe Gly Glu Asp Thr Ser Ala Leu Pro Ala Pro Gly Gly Gly
   625                 630                 635                 640
   Gly Ser Gly Gly Asp Pro Thr
                   645
<210> 47
   <211> 712
   <212> PRT
   <213> human
<400> 47
   Met Glu Asn Leu Gly Val Gly Glu Gly Ala Glu Ala Cys Ser Arg Leu
    1               5                  10                  15
   Ser Arg Ser Arg Gly Arg His Ser Met Thr Arg Ala Pro Lys His Leu
               20                  25                  30
   Trp Arg Gln Pro Arg Arg Pro Ile Arg Ile Gln Gln Arg Phe Tyr Ser
           35                  40                  45
   Asp Pro Asp Lys Ser Ala Gly Cys Arg Glu Arg Asp Leu Ser Pro Arg
       50                  55                  60
   Pro Glu Leu Arg Lys Ser Arg Leu Ser Trp Pro Val Ser Ser Cys Arg
   65                  70                  75                  80
   Arg Phe Asp Leu Glu Asn Gly Leu Ser Cys Gly Arg Arg Ala Leu Asp
                   85                  90                  95
   Pro Gln Ser Ser Pro Gly Leu Gly Arg Ile Met Gln Ala Pro Val Pro
               100                 105                 110
   His Ser Gln Arg Arg Glu Ser Phe Leu Tyr Arg Ser Asp Ser Asp Tyr
           115                 120                 125
   Glu Leu Ser Pro Lys Ala Met Ser Arg Asn Ser Ser Val Ala Ser Asp
       130                 135                 140
   Leu His Gly Glu Asp Met Ile Val Thr Pro Phe Ala Gln Val Leu Ala
   145                 150                 155                 160
   Ser Leu Arg Thr Val Arg Ser Asn Val Ala Ala Leu Ala Arg Gln Gln
                   165                 170                 175
   Cys Leu Gly Ala Ala Lys Gln Gly Pro Val Gly Asn Pro Ser Ser Ser
               180                 185                 190
   Asn Gln Leu Pro Pro Ala Glu Asp Thr Gly Gln Lys Leu Ala Leu Glu
           195                 200                 205
   Thr Leu Asp Glu Leu Asp Trp Cys Leu Asp Gln Leu Glu Thr Leu Gln
       210                 215                 220
   Thr Arg His Ser Val Gly Glu Met Ala Ser Asn Lys Phe Lys Arg Ile
   225                 230                 235                 240
   Leu Asn Arg Glu Leu Thr His Leu Ser Glu Thr Ser Arg Ser Gly Asn
                   245                 250                 255
   Gln Val Ser Glu Tyr Ile Ser Arg Thr Phe Leu Asp Gln Gln Thr Glu
               260                 265                 270
   Val Glu Leu Pro Lys Val Thr Ala Glu Glu Ala Pro Gln Pro Met Ser
           275                 280                 285
   Arg Ile Ser Gly Leu His Gly Leu Cys His Ser Ala Ser Leu Ser Ser
       290                 295                 300
   Ala Thr Val Pro Arg Phe Gly Val Gln Thr Asp Gln Glu Glu Gln Leu
   305                 310                 315                 320
   Ala Lys Glu Leu Glu Asp Thr Asn Lys Trp Gly Leu Asp Val Phe Lys
                   325                 330                 335
   Val Ala Asp Val Ser Gly Asn Arg Pro Leu Thr Ala Ile Ile Phe Ser
               340                 345                 350
   Ile Phe Gln Glu Arg Asp Leu Leu Lys Thr Phe Gln Ile Pro Ala Asp
           355                 360                 365
   Thr Leu Ala Thr Tyr Leu Leu Met Leu Glu Gly His Tyr His Ala Asn
       370                 375                 380
   Val Ala Tyr His Asn Ser Leu His Ala Ala Asp Val Ala Gln Ser Thr
   385                 390                 395                 400
   His Val Leu Leu Ala Thr Pro Ala Leu Glu Ala Val Phe Thr Asp Leu
                   405                 410                 415
   Glu Ile Leu Ala Ala Leu Phe Ala Ser Ala Ile His Asp Val Asp His
               420                 425                 430
   Pro Gly Val Ser Asn Gln Phe Leu Ile Asn Thr Asn Ser Asp Val Ala
           435                 440                 445
   Leu Met Tyr Asn Asp Ala Ser Val Leu Glu Asn His His Leu Ala Val
       450                 455                 460
   Gly Phe Lys Leu Leu Gln Ala Glu Asn Cys Asp Ile Phe Gln Asn Leu
   465                 470                 475                 480
   Ser Ala Lys Gln Arg Leu Ser Leu Arg Arg Met Val Ile Asp Met Val
                   485                 490                 495
   Leu Ala Thr Asp Met Ser Lys His Met Asn Leu Leu Ala Asp Leu Lys
               500                 505                 510
   Thr Met Val Glu Thr Lys Lys Val Thr Ser Leu Gly Val Leu Leu Leu
           515                 520                 525
   Asp Asn Tyr Ser Asp Arg Ile Gln Val Leu Gln Asn Leu Val His Cys
       530                 535                 540
   Ala Asp Leu Ser Asn Pro Thr Lys Pro Leu Pro Leu Tyr Arg Gln Trp
   545                 550                 555                 560
   Thr Asp Arg Ile Met Ala Glu Phe Phe Gln Gln Gly Asp Arg Glu Arg
                   565                 570                 575
   Glu Ser Gly Leu Asp Ile Ser Pro Met Cys Asp Lys His Thr Ala Ser
               580                 585                 590
   Val Glu Lys Ser Gln Val Gly Phe Ile Asp Tyr Ile Ala His Pro Leu
           595                 600                 605
   Trp Glu Thr Trp Ala Asp Leu Val His Pro Asp Ala Gln Asp Leu Leu
       610                 615                 620
   Asp Thr Leu Glu Asp Asn Arg Glu Trp Tyr Gln Ser Lys Ile Pro Arg
   625                 630                 635                 640
   Ser Pro Ser Asp Leu Thr Asn Pro Glu Arg Asp Gly Pro Asp Arg Phe
                   645                 650                 655
   Gln Phe Glu Leu Thr Leu Glu Glu Ala Glu Glu Glu Asp Glu Glu Glu
               660                 665                 670
   Glu Glu Glu Gly Glu Glu Thr Ala Leu Ala Lys Glu Ala Leu Glu Leu
           675                 680                 685
   Pro Asp Thr Glu Leu Leu Ser Pro Glu Ala Gly Pro Asp Pro Gly Asp
       690                 695                 700
   Leu Pro Leu Asp Asn Gln Arg Thr
   705                 710
<210> 48
   <211> 564
   <212> PRT
   <213> human
<400> 48
   Met Lys Glu His Gly Gly Thr Phe Ser Ser Thr Gly Ile Ser Gly Gly
    1               5                  10                  15
   Ser Gly Asp Ser Ala Met Asp Ser Leu Gln Pro Leu Gln Pro Asn Tyr
               20                  25                  30
   Met Pro Val Cys Leu Phe Ala Glu Glu Ser Tyr Gln Lys Leu Ala Met
           35                  40                  45
   Glu Thr Leu Glu Glu Leu Asp Trp Cys Leu Asp Gln Leu Glu Thr Ile
       50                  55                  60
   Gln Thr Tyr Arg Ser Val Ser Glu Met Ala Ser Asn Lys Phe Lys Arg
   65                  70                  75                  80
   Met Leu Asn Arg Glu Leu Thr His Leu Ser Glu Met Ser Arg Ser Gly
                   85                  90                  95
   Asn Gln Val Ser Glu Tyr Ile Ser Asn Thr Phe Leu Asp Lys Gln Asn
               100                 105                 110
   Asp Val Glu Ile Pro Ser Pro Thr Gln Lys Asp Arg Glu Lys Lys Lys
           115                 120                 125
   Lys Gln Gln Leu Met Thr Gln Ile Ser Gly Val Lys Lys Leu Met His
       130                 135                 140
   Ser Ser Ser Leu Asn Asn Thr Ser Ile Ser Arg Phe Gly Val Asn Thr
   145                 150                 155                 160
   Glu Asn Glu Asp His Leu Ala Lys Glu Leu Glu Asp Leu Asn Lys Trp
                   165                 170                 175
   Gly Leu Asn Ile Phe Asn Val Ala Gly Tyr Ser His Asn Arg Pro Leu
               180                 185                 190
   Thr Cys Ile Met Tyr Ala Ile Phe Gln Glu Arg Asp Leu Leu Lys Thr
           195                 200                 205
   Phe Arg Ile Ser Ser Asp Thr Phe Ile Thr Tyr Met Met Thr Leu Glu
       210                 215                 220
   Asp His Tyr His Ser Asp Val Ala Tyr His Asn Ser Leu His Ala Ala
   225                 230                 235                 240
   Asp Val Ala Gln Ser Thr His Val Leu Leu Ser Thr Pro Ala Leu Asp
                   245                 250                 255
   Ala Val Phe Thr Asp Leu Glu Ile Leu Ala Ala Ile Phe Ala Ala Ala
               260                 265                 270
   Ile His Asp Val Asp His Pro Gly Val Ser Asn Gln Phe Leu Ile Asn
           275                 280                 285
   Thr Asn Ser Glu Leu Ala Leu Met Tyr Asn Asp Glu Ser Val Leu Glu
       290                 295                 300
   Asn His His Leu Ala Val Gly Phe Lys Leu Leu Gln Glu Glu His Cys
   305                 310                 315                 320
   Asp Ile Phe Met Asn Leu Thr Lys Lys Gln Arg Gln Thr Leu Arg Lys
                   325                 330                 335
   Met Val Ile Asp Met Val Leu Ala Thr Asp Met Ser Lys His Met Ser
               340                 345                 350
   Leu Leu Ala Asp Leu Lys Thr Met Val Glu Thr Lys Lys Val Thr Ser
           355                 360                 365
   Ser Gly Val Leu Leu Leu Asp Asn Tyr Thr Asp Arg Ile Gln Val Leu
       370                 375                 380
   Arg Asn Met Val His Cys Ala Asp Leu Ser Asn Pro Thr Lys Ser Leu
   385                 390                 395                 400
   Glu Leu Tyr Arg Gln Trp Thr Asp Arg Ile Met Glu Glu Phe Phe Gln
                   405                 410                 415
   Gln Gly Asp Lys Glu Arg Glu Arg Gly Met Glu Ile Ser Pro Met Cys
               420                 425                 430
   Asp Lys His Thr Ala Ser Val Glu Lys Ser Gln Val Gly Phe Ile Asp
           435                 440                 445
   Tyr Ile Val His Pro Leu Trp Glu Thr Trp Ala Asp Leu Val Gln Pro
       450                 455                 460
   Asp Ala Gln Asp Ile Leu Asp Thr Leu Glu Asp Asn Arg Asn Trp Tyr
   465                 470                 475                 480
   Gln Ser Met Ile Pro Gln Ser Pro Ser Pro Pro Leu Asp Glu Gln Asn
                   485                 490                 495
   Arg Asp Cys Gln Gly Leu Met Glu Lys Phe Gln Phe Glu Leu Thr Leu
               500                 505                 510
   Asp Glu Glu Asp Ser Glu Gly Pro Glu Lys Glu Gly Glu Gly His Ser
           515                 520                 525
   Tyr Phe Ser Ser Thr Lys Thr Leu Cys Val Ile Asp Pro Glu Asn Arg
       530                 535                 540
   Asp Ser Leu Gly Glu Thr Asp Ile Asp Ile Ala Thr Glu Asp Lys Ser
   545                 550                 555                 560
   Pro Val Asp Thr
<210> 49
   <211> 507
   <212> PRT
   <213> human
<400> 49
<210> 50
   <211> 745
   <212> PRT
   <213> human
<400> 50
   Met Ala Gln Gln Thr Ser Pro Asp Thr Leu Thr Val Pro Glu Val Asp
    1               5                  10                  15
   Asn Pro His Cys Pro Asn Pro Trp Leu Asn Glu Asp Leu Val Lys Ser
               20                  25                  30
   Leu Arg Glu Asn Leu Leu Gln His Glu Lys Ser Lys Thr Ala Arg Lys
           35                  40                  45
   Ser Val Ser Pro Lys Leu Ser Pro Val Ile Ser Pro Arg Asn Ser Pro
       50                  55                  60
   Arg Leu Leu Arg Arg Met Leu Leu Ser Ser Asn Ile Pro Lys Gln Arg
   65                  70                  75                  80
   Arg Phe Thr Val Ala His Thr Cys Phe Asp Val Asp Asn Gly Thr Ser
                   85                  90                  95
   Ala Gly Arg Ser Pro Leu Asp Pro Met Thr Ser Pro Gly Ser Gly Leu
               100                 105                 110
   Ile Leu Gln Ala Asn Phe Val His Ser Gln Arg Arg Glu Ser Phe Leu
           115                 120                 125
   Tyr Arg Ser Asp Ser Asp Tyr Asp Leu Ser Pro Lys Ser Met Ser Arg
       130                 135                 140
   Asn Ser Ser Ile Ala Ser Asp Ile His Gly Asp Asp Leu Ile Val Thr
   145                 150                 155                 160
   Pro Phe Ala Gln Val Leu Ala Ser Leu Arg Thr Val Arg Asn Asn Phe
                   165                 170                 175
   Ala Ala Leu Thr Asn Leu Gln Asp Arg Ala Pro Ser Lys Arg Ser Pro
               180                 185                 190
   Met Cys Asn Gln Pro Ser Ile Asn Lys Ala Thr Ile Thr Glu Glu Ala
           195                 200                 205
   Tyr Gln Lys Leu Ala Ser Glu Thr Leu Glu Glu Leu Asp Trp Cys Leu
       210                 215                 220
   Asp Gln Leu Glu Thr Leu Gln Thr Arg His Ser Val Ser Glu Met Ala
   225                 230                 235                 240
   Ser Asn Lys Phe Lys Arg Met Leu Asn Arg Glu Leu Thr His Leu Ser
                   245                 250                 255
   Glu Met Ser Arg Ser Gly Asn Gln Val Ser Glu Phe Ile Ser Asn Thr
               260                 265                 270
   Phe Leu Asp Lys Gln His Glu Val Glu Ile Pro Ser Pro Thr Gln Lys
           275                 280                 285
   Glu Lys Glu Lys Lys Lys Arg Pro Met Ser Gln Ile Ser Gly Val Lys
       290                 295                 300
   Lys Leu Met His Ser Ser Ser Leu Thr Asn Ser Ser Ile Pro Arg Phe
   305                 310                 315                 320
   Gly Val Lys Thr Glu Gln Glu Asp Val Leu Ala Lys Glu Leu Glu Asp
                   325                 330                 335
   Val Asn Lys Trp Gly Leu His Val Phe Arg Ile Ala Glu Leu Ser Gly
               340                 345                 350
   Asn Arg Pro Leu Thr Val Ile Met His Thr Ile Phe Gln Glu Arg Asp
           355                 360                 365
   Leu Leu Lys Thr Phe Lys Ile Pro Val Asp Thr Leu Ile Thr Tyr Leu
       370                 375                 380
   Met Thr Leu Glu Asp His Tyr His Ala Asp Val Ala Tyr His Asn Asn
   385                 390                 395                 400
   Ile His Ala Ala Asp Val Val Gln Ser Thr His Val Leu Leu Ser Thr
                   405                 410                 415
   Pro Ala Leu Glu Ala Val Phe Thr Asp Leu Glu Ile Leu Ala Ala Ile
               420                 425                 430
   Phe Ala Ser Ala Ile His Asp Val Asp His Pro Gly Val Ser Asn Gln
           435                 440                 445
   Phe Leu Ile Asn Thr Asn Ser Glu Leu Ala Leu Met Tyr Asn Asp Ser
       450                 455                 460
   Ser Val Leu Glu Asn His His Leu Ala Val Gly Phe Lys Leu Leu Gln
   465                 470                 475                 480
   Glu Glu Asn Cys Asp Ile Phe Gln Asn Leu Thr Lys Lys Gln Arg Gln
                   485                 490                 495
   Ser Leu Arg Lys Met Val Ile Asp Ile Val Leu Ala Thr Asp Met Ser
               500                 505                 510
   Lys His Met Asn Leu Leu Ala Asp Leu Lys Thr Met Val Glu Thr Lys
           515                 520                 525
   Lys Val Thr Ser Ser Gly Val Leu Leu Leu Asp Asn Tyr Ser Asp Arg
       530                 535                 540
   Ile Gln Val Leu Gln Asn Met Val His Cys Ala Asp Leu Ser Asn Pro
   545                 550                 555                 560
   Thr Lys Pro Leu Gln Leu Tyr Arg Gln Trp Thr Asp Arg Ile Met Glu
                   565                 570                 575
   Glu Phe Phe Arg Gln Gly Asp Arg Glu Arg Glu Arg Gly Met Glu Ile
               580                 585                 590
   Ser Pro Met Cys Asp Lys His Asn Ala Ser Val Glu Lys Ser Gln Val
           595                 600                 605
   Gly Phe Ile Asp Tyr Ile Val His Pro Leu Trp Glu Thr Trp Ala Asp
       610                 615                 620
   Leu Val His Pro Asp Ala Gln Asp Ile Leu Asp Thr Leu Glu Asp Asn
   625                 630                 635                 640
   Arg Glu Trp Tyr Gln Ser Thr Ile Pro Gln Ser Pro Ser Pro Ala Pro
                   645                 650                 655
   Asp Asp Pro Glu Glu Gly Arg Gln Gly Gln Thr Glu Lys Phe Gln Phe
               660                 665                 670
   Glu Leu Thr Leu Glu Glu Asp Gly Glu Ser Asp Thr Glu Lys Asp Ser
           675                 680                 685
   Gly Ser Gln Val Glu Glu Asp Thr Ser Cys Ser Asp Ser Lys Thr Leu
       690                 695                 700
   Cys Thr Gln Asp Ser Glu Ser Thr Glu Ile Pro Leu Asp Glu Gln Val
   705                 710                 715                 720
   Glu Glu Glu Ala Val Gly Glu Glu Glu Glu Ser Gln Pro Glu Ala Cys
                   725                 730                 735
   Val Ile Asp Asp Arg Ser Pro Asp Thr
               740                 745
<210> 51
   <211> 673
   <212> PRT
   <213> human
<400> 51
   Met Met His Val Asn Asn Phe Pro Phe Arg Arg His Ser Trp Ile Cys
    1               5                  10                  15
   Phe Asp Val Asp Asn Gly Thr Ser Ala Gly Arg Ser Pro Leu Asp Pro
               20                  25                  30
   Met Thr Ser Pro Gly Ser Gly Leu Ile Leu Gln Ala Asn Phe Val His
           35                  40                  45
   Ser Gln Arg Arg Glu Ser Phe Leu Tyr Arg Ser Asp Ser Asp Tyr Asp
       50                  55                  60
   Leu Ser Pro Lys Ser Met Ser Arg Asn Ser Ser Ile Ala Ser Asp Ile
   65                  70                  75                  80
   His Gly Asp Asp Leu Ile Val Thr Pro Phe Ala Gln Val Leu Ala Ser
                   85                  90                  95
   Leu Arg Thr Val Arg Asn Asn Phe Ala Ala Leu Thr Asn Leu Gln Asp
               100                 105                 110
   Arg Ala Pro Ser Lys Arg Ser Pro Met Cys Asn Gln Pro Ser Ile Asn
           115                 120                 125
   Lys Ala Thr Ile Thr Glu Glu Ala Tyr Gln Lys Leu Ala Ser Glu Thr
       130                 135                 140
   Leu Glu Glu Leu Asp Trp Cys Leu Asp Gln Leu Glu Thr Leu Gln Thr
   145                 150                 155                 160
   Arg His Ser Val Ser Glu Met Ala Ser Asn Lys Phe Lys Arg Met Leu
                   165                 170                 175
   Asn Arg Glu Leu Thr His Leu Ser Glu Met Ser Arg Ser Gly Asn Gln
               180                 185                 190
   Val Ser Glu Phe Ile Ser Asn Thr Phe Leu Asp Lys Gln His Glu Val
           195                 200                 205
   Glu Ile Pro Ser Pro Thr Gln Lys Glu Lys Glu Lys Lys Lys Arg Pro
       210                 215                 220
   Met Ser Gln Ile Ser Gly Val Lys Lys Leu Met His Ser Ser Ser Leu
   225                 230                 235                 240
   Thr Asn Ser Ser Ile Pro Arg Phe Gly Val Lys Thr Glu Gln Glu Asp
                   245                 250                 255
   Val Leu Ala Lys Glu Leu Glu Asp Val Asn Lys Trp Gly Leu His Val
               260                 265                 270
   Phe Arg Ile Ala Glu Leu Ser Gly Asn Arg Pro Leu Thr Val Ile Met
           275                 280                 285
   His Thr Ile Phe Gln Glu Arg Asp Leu Leu Lys Thr Phe Lys Ile Pro
       290                 295                 300
   Val Asp Thr Leu Ile Thr Tyr Leu Met Thr Leu Glu Asp His Tyr His
   305                 310                 315                 320
   Ala Asp Val Ala Tyr His Asn Asn Ile His Ala Ala Asp Val Val Gln
                   325                 330                 335
   Ser Thr His Val Leu Leu Ser Thr Pro Ala Leu Glu Ala Val Phe Thr
               340                 345                 350
   Asp Leu Glu Ile Leu Ala Ala Ile Phe Ala Ser Ala Ile His Asp Val
           355                 360                 365
   Asp His Pro Gly Val Ser Asn Gln Phe Leu Ile Asn Thr Asn Ser Glu
       370                 375                 380
   Leu Ala Leu Met Tyr Asn Asp Ser Ser Val Leu Glu Asn His His Leu
   385                 390                 395                 400
   Ala Val Gly Phe Lys Leu Leu Gln Glu Glu Asn Cys Asp Ile Phe Gln
                   405                 410                 415
   Asn Leu Thr Lys Lys Gln Arg Gln Ser Leu Arg Lys Met Val Ile Asp
               420                 425                 430
   Ile Val Leu Ala Thr Asp Met Ser Lys His Met Asn Leu Leu Ala Asp
           435                 440                 445
   Leu Lys Thr Met Val Glu Thr Lys Lys Val Thr Ser Ser Gly Val Leu
       450                 455                 460
   Leu Leu Asp Asn Tyr Ser Asp Arg Ile Gln Val Leu Gln Asn Met Val
   465                 470                 475                 480
   His Cys Ala Asp Leu Ser Asn Pro Thr Lys Pro Leu Gln Leu Tyr Arg
                   485                 490                 495
   Gln Trp Thr Asp Arg Ile Met Glu Glu Phe Phe Arg Gln Gly Asp Arg
               500                 505                 510
   Glu Arg Glu Arg Gly Met Glu Ile Ser Pro Met Cys Asp Lys His Asn
           515                 520                 525
   Ala Ser Val Glu Lys Ser Gln Val Gly Phe Ile Asp Tyr Ile Val His
       530                 535                 540
   Pro Leu Trp Glu Thr Trp Ala Asp Leu Val His Pro Asp Ala Gln Asp
   545                 550                 555                 560
   Ile Leu Asp Thr Leu Glu Asp Asn Arg Glu Trp Tyr Gln Ser Thr Ile
                   565                 570                 575
   Pro Gln Ser Pro Ser Pro Ala Pro Asp Asp Pro Glu Glu Gly Arg Gln
               580                 585                 590
   Gly Gln Thr Glu Lys Phe Gln Phe Glu Leu Thr Leu Glu Glu Asp Gly
           595                 600                 605
   Glu Ser Asp Thr Glu Lys Asp Ser Gly Ser Gln Val Glu Glu Asp Thr
       610                 615                 620
   Ser Cys Ser Asp Ser Lys Thr Leu Cys Thr Gln Asp Ser Glu Ser Thr
   625                 630                 635                 640
   Glu Ile Pro Leu Asp Glu Gln Val Glu Glu Glu Ala Val Gly Glu Glu
                   645                 650                 655
   Glu Glu Ser Gln Pro Glu Ala Cys Val Ile Asp Asp Arg Ser Pro Asp
               660                 665                 670
   Thr

## Description

### BACKGROUND OF THE INVENTION

The enzyme phosphodiesterase (PDE), along with adenylate cyclase and guanylate cyclase, are enzymes responsible for maintaining the correct balance of cyclic AMP and cyclic GMP in cells. There are multiple distinct phosphodiesterases (PDE 1 through PDE 9), most of which exist as two or more isozymes or splice variants that can differ in their cellular distribution, specificity toward hydrolysis of cAMP or cGMP, selective inhibition by various compounds, and sensitivity to regulation by calcium, calmodulin, cAMP, and cGMP (J.A. Beavo in Cyclic Nucleotide Phosphodiesterases: Structure, Regulation and Drug Action. Multiple Phosphodiesterase Isozymes: Background, Nomenclature, and Implications. Eds. Beavo, J. and Houslay, M.D., John Wiley and Son, New York, 1990, pp. 3-15 and T.J. Torphy et al., "Novel Phosphodiesterases Inhibitors for the Therapy of Asthma", Drug News & Prospective, 6(4) May 1993, pp. 203-214). The PDE4 family, which is specific for cAMP, is composed of at least 4 isozymes (a-d), and multiple splice variants (Houslay, M.D., et al. in Advances in Pharmacology 44, Eds. J. August et al., p.225, 1998). In total, there may be over 20 PDE4 isoforms expressed in a cell specific pattern regulated by a number of different promoters.

PDE4 is present in the brain and major inflammatory cells and has been found in abnormally elevated levels in a number of diseases including atopic dermatitis or eczema, asthma, and hay fever *(*ASTI Connections, Vol. 8 #1 (1996) p. 3 and J. of Allergy and Clinical Immunology 70:452-457,1982). Disease states for which selective PDE4 inhibitors have been sought include: asthma, atopic dermatitis, depression, reperfusion injury, septic shock, toxic shock, endotoxic shock, adult respiratory distress syndrome, autoimmune diabetes, diabetes insipidus, multi-infarct dementia, AIDS, cancer, Crohn's disease, multiple sclerosis, cerebral ischemia, psoriasis, allograft rejection, restenosis, ulcerative colitis, cachexia, cerebral malaria, allergic rhino-conjunctivitis, osteoarthritis, rheumatoid arthritis, chronic bronchitis, eosinophilic granuloma, and autoimmune encephalomyelitis *(Houslay et al.,* 1998). In individuals suffering from atopic diseases, elevated PDE4 activity can be detected in their peripheral blood mononuclear leukocytes, T cells, mast cells, neutrophils and basophils. This increased PDE activity decreases cAMP levels and results in a breakdown of cAMP control in these cells, which in turn results in increased immune response in the blood and tissues of affected individuals.

PDE inhibitors influence multiple functional pathways, act on multiple immune and inflammatory pathways, and influence synthesis or release of numerous immune mediators (J.M. Hanifin and S.C. Chan, "Atopic Dermatitis-Therapeutic Implication for New Phosphodiesterase Inhibitors, Monocyte Dysregulation of T Cells" in AACI News, 7/2, 1995; J.M. Hanifin et al., "Type 4 Phosphodiesterase Inhibitors Have Clinical and In Vitro Anti-inflammatory Effects in Atopic Dermatitis," J. of Invest. Derm., 1996, 107:51-56 and Cohen, V.L. in INC's 7th Annual Conference on Asthma and Allergy (Oct. 27-28, 1997) - Phosphodiesterase 4 Inhibitors: Second Generation and Beyond*).* Clinical use of inhibitors of PDE4 have shown them to be broad spectrum anti-inflammatory agents with impressive activity in models of asthma and other allergic disorders, including atopic dermatitis and hay fever. PDE4 inhibitors that have been used clinically include theophylline, rolipram, denbufylline, CDP 840 (a tri-aryl ethane) and CP80633 (a pyrimidone). PDE4 inhibitors have been shown to influence eosinophil responses, decrease basophil histamine release, decrease IgE, PGE2, and IL 10 synthesis, and decrease anti-CD3 stimulated IL4 production.

All of the PDE4 inhibitors developed to date have been small molecule compounds which exhibit central nervous system and gastrointestinal effects. Although the first generation of PDE4 inhibitors have been effective in reducing PDE4 activity and alleviating a number of the inflammatory problems caused by overexpression of this enzyme, their effectiveness has been limited by mechanism related side effects and none of them can be used systemically because of nausea and vomiting *(*ASTI Connections, Vol. 8 #1 (1996) p. 3 and Hanifin reference and Cohen, V.L. in INC's 7th Annual Conference on Asthma and Allergy (Oct. 27-28, 1997) - Phosphodiesterase 4 Inhibitors: Second Generation and Beyond*).*

Oligonucleotide therapy, i.e., the use of oligonucleotides to modulate the expression of specific genes, offers an opportunity to selectively modify the expression of genes without the undesirable non-specific toxic effects of more traditional therapeutics. The use of antisense oligonucleotides has emerged as a powerful new approach for the treatment of many diseases. The preponderance of the work to date has focused on the use of antisense oligonucleotides as antiviral agents or as anticancer agents (Wickstrom, E., Ed., Prospects for Antisense Nucleic Acid Therapy of Cancer. and AIDS, New York: Wiley-Liss, 1991; Crooke, S.T., and Lebleu, B., Eds., Antisense Research and Applications, Boca Raton: CRC Press, 1993, pp. 154-182; Baserga, R., and Denhardt, D.T., 1992, Antisense Strategies, New York: The New York Academy of Sciences, Vol. 660; Murray, J.A.H., Ed., Antisense RNA and DNA, New York: Wiley-Liss, 1993, Agrawal et al., Proc. Natl. Acad Sci. USA 85:7079-7083 (1988), Zamecnik et al., Proc. Natl. Acad Sci. USA 83:4143-4146 (1986)).

There is a need to develop a therapeutic agent for modulating PDE4 that is effective but does not possess the side effects of the current PDE4 inhibitors. There is thus a need for a bioavailable, non-toxic therapeutic agent that specifically modulates PDE4.

### SUMMARY OF THE INVENTION

The present invention provides end-blocked acid resistant nucleic acid polymers, e.g., end-blocked 2'-O-alkyl and 2'-O-alkyl-n(O-alkyl) oligonucleotides, for use in modulating PDE4D activity, either *in vivo* or *in vitro.* These PDE4D polymers exhibit substantial stability at low pH, substantial resistance to nuclease degradation, and binding specificity both *in vivo* and *in vitro.* The nucleotides of these polymers may be either ribonucleotides and/or deoxyribonucleotides, and may be targeted to DNA sequences involved in the expression of PDE4D (e.g., coding sequences, promoter sequences, enhancer sequences, etc.) or to PDE4D mRNA. These low toxicity, highly specific, acid stable, end-blocked polymers represent an improved nucleic acid structure for therapeutic treatments of PDE4D-mediated diseases. The 3', 5', or 3' and 5' acid stable, nuclease resistant ends confer improved bioavailability by increasing nuclease resistance.

The invention also provides pharmaceutical compositions comprised of polymers of the invention. These pharmaceutical compositions may include any pharmaceutically acceptable carrier. The pharmaceutical composition may also include additives such as adjuvants, stabilizers, fillers and the like.

In the invention also provided are the acid resistant polymers comprising a nucleic acid having a backbone structure modified from that of a naturally occurring nucleotide polymer ; and a blocking chemical modification at or near at least one end of the nucleic acid used for treating a patient in need of such treatment with a therapeutically effective amount of an polymers targeted to PDE4D. The therapeutic effectiveness of an oligonucleotide targeted against PDE4D is demonstrated herein using data from preliminary human trials.

The invention also comprises methods of reducing the activity of one or more PDE4D enzyme comprising treating a patient with one or more antisense oligonucleotide polymers. Preferably the oligonucleotide is targeted to mRNA coding for PDE4D.

It is an advantage of the polymers of the invention that the acid stable ends confer an improved stability on the modified nucleic acids in an acidic environment (e.g., the stomach, with a pH of 1 to 2), and thus increase bioavailability of the polymers *in vivo.*

It is another advantage of the polymers of the invention that they bind with specificity to PDE4 target sequences *in vivo* and *in vitro.*

It is another advantage of the invention that the end blocked nucleic acids are non-toxic to a subject treated with the modified nucleic acids. The modified nucleic acids of the present invention, e.g., 2'-O-alkyl and 2'-O-alkyl-n(O-alkyl) end-blocked oligonucleotides, do not display side effects commonly caused by therapeutic administration of regular polyanionic oligonucleotides, such as increased binding to serum and other proteins, stimulation of serum transaminases, decreases in platelet counts, and the like.

It is yet another advantage that the acid stable ends confer an improved stability on the PDE4D oligonucleotides in the acid environments of lysosomal vesicles in macrophages and neutrophils.

It is a further advantage that the PDE4D oligonucleotides of the present invention are readily encapsulated in charged liposomes.

It is a further advantage that the PDE4D polymers have low toxicity, i.e., mice parenterally treated with a PDE4D oligonucleotide of the invention exhibit an LD₅₀ of less than one at 400 mg/ml.

These and other objects, advantages, and features of the invention will become apparent to those skilled in the art upon reading the details of the nucleic acids and uses thereof as more fully described below.

### DETAILED DESCRIPTION OF PREFERRED EMBODIMENTS

Before the present invention is described, it is to be understood that this invention is not limited to the particular methodology, protocols, cell lines, animal species or genera, constructs, and reagents described, as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "a construct" includes a plurality of such constructs and reference to "the oligonucleotide" includes reference to one or more oligonucleotides and equivalents thereof known to those skilled in the art, and so forth.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing, for example, the cell lines, constructs, and methodologies that are described in the publications which might be used in connection with the presently described invention. The publications discussed above and throughout the text are provided solely for their disclosure prior to the filing date of the present application. Nothing herein is to be construed as an admission that the inventors are not entitled to antedate such disclosure by virtue of prior invention.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

The terms "nucleic acid" and "nucleic acid molecule" as used interchangeably herein, refer to a molecule comprised of nucleotides, i.e., ribonucleotides, deoxyribonucleotides, or both. The term includes monomers and polymers of ribonucleotides and deoxyribonucleotides, with the ribonucleotide and/or deoxyribonucleotides being connected together, in the case of the polymers, via 5' to 3' linkages. However, linkages may include any of the linkages known in the nucleic acid synthesis art including, for example, nucleic acids comprising 5' to 2' linkages. The nucleotides used in the nucleic acid molecule may be naturally occurring or may be synthetically produced analogues that are capable of forming base-pair relationships with naturally occurring base pairs. Examples of non-naturally occurring bases that are capable of forming base-pairing relationships include, but are not limited to, aza and deaza pyrimidine analogues, aza and deaza purine analogues, and other heterocyclic base analogues, wherein one or more of the carbon and nitrogen atoms of the purine and pyrimidine rings have been substituted by heteroatoms, e.g., oxygen, sulfur, selenium, phosphorus, and the like.

The term "oligonucleotide" as used herein refers to a nucleic acid molecule comprising nucleotides, in a number as given in the claimed sequences.

The term PDE4D oligonucleotide" as used herein refers to an oligonucleotide that is targeted to sequences that affect PDE4D expression or activity. These include, but are not limited to, PDE4D DNA coding sequences, PDE4D DNA promoter sequences, PDE4D DNA enhancer sequences, mRNA encoding PDE4D and the like.

The terms "modified oligonucleotide" and "modified nucleic acid molecule" as used herein refer to nucleic acids, including oligonucleotides, with one or more chemical modifications at the molecular level of the natural molecular structures of all or any of the nucleic acid bases, sugar moieties, internucleoside phosphate linkages, as well as molecules having added substituents, such as diamines, cholesteryl or other lipophilic groups, or a combination of modifications at these sites. The internucleoside phosphate linkages can be phosphodiester, phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate and/or sulfone internucleotide linkages, or 3'-3', 2'-5' or 5'-5' linkages, and combinations of such similar linkages (to produce mixed backbone modified oligonucleotides). The modifications can be internal (single or repeated) or at the end(s) of the oligonucleotide molecule and can include additions to the molecule of the internucleoside phosphate linkages, such as cholesteryl, diamine compounds with varying numbers of carbon residues between amino groups and terminal ribose, deoxyribose and phosphate modifications which cleave or crosslink to the opposite chains or to associated enzymes or other proteins. Electrophilic groups such as ribose-dialdehyde could covalently link with an epsilon amino group of the lysyl-residue of such a protein. A nucleophilic group such as n-ethylmaleimide tethered to an oligomer could covalently attach to the 5' end of an mRNA or to another electrophilic site. The term modified oligonucleotides also includes oligonucleotides comprising modifications to the sugar moieties such as 2'-substituted ribonucleotides, or deoxyribonucleotide monomers, any of which are connected together via 5' to 3' linkages. Modified oligonucleotides may also be comprised of PNA or morpholino modified backbones where target specificity of the sequence is maintained.

The term "nucleic acid backbone" as used herein refers to the structure of the chemical moiety linking nucleotides in a molecule. This may include structures formed from any and all means of chemically linking nucleotides. A modified backbone as used herein includes modifications to the chemical linkage between nucleotides, as well as other modifications that may be used to enhance stability and affinity, such as modifications to the sugar structure. For example an a-anomer of deoxyribose may be used, where the base is inverted with respect to the natural b-anomer. In a preferred embodiment, the 2'-OH of the sugar group may be altered to 2'-O-alkyl or 2'-O-alkyl-n(O-alkyl), which provides resistance to degradation without comprising affinity.

The term "acidification" and "protonation/acidification" as used interchangeably herein refers to the process by which protons (or positive hydrogen ions) are added to proton acceptor sites on a nucleic acid. The proton acceptor sites include the amine groups on the base structures of the nucleic acid and the phosphate of the phosphodiester linkages. As the pH is decreased, the number of these acceptor sites which are protonated increases, resulting in a more highly protonated/acidified nucleic acid.

The term "protonated/acidified nucleic acid" refers to a nucleic acid that, when dissolved in water at a concentration of approximately 16 A₂₆₀ per ml, has a pH lower than physiological pH, i.e., lower than approximately pH 7. Modified nucleic acids, nuclease-resistant nucleic acids, and antisense nucleic acids are meant to be encompassed by this definition. Generally, nucleic acids are protonated/acidified by adding protons to the reactive sites on a nucleic acid, although other modifications that will decrease the pH of the nucleic acid can also be used and are intended to be encompassed by this term.

The term "end-blocked" as used herein refers to a nucleic acid with a chemical modification at the molecular level that prevents the degradation of selected nucleotides, e.g., by nuclease action. This chemical modification is positioned such that it protects the integral portion of the nucleic acid, for example the coding region of an antisense oligonucleotide. An end block may be a 3' end block or a 5' end block. For example, a 3' end block may be at the 3'-most position of the molecule, or it may be internal to the 3' ends, provided it is 3' of the integral sequences of the nucleic acid.

The term "substantially nuclease resistant" refers to nucleic acids that are resistant to nuclease degradation, as compared to naturally occurring or unmodified nucleic acids. Modified nucleic acids of the invention are at least 1.25 times more resistant to nuclease degradation than their unmodified counterpart, more preferably at least 2 times more resistant, even more preferably at least 5 times more resistant, and most preferably at least 10 times more resistant than their unmodified counterpart. Such substantially nuclease resistant nucleic acids include, but are not limited to, nucleic acids with modified backbones such as 2'-O-methyl, 2'-0-alkyl, 2'-O-alkyl-n(O-alkyl).

The term "substantially acid resistant" as used herein refers to nucleic acids that are resistant to acid degradation as compared to unmodified nucleic acids. Typically, the relative acid resistance of a nucleic acid will be measured by comparing the percent degradation of a resistant nucleic acid with the percent degradation of its unmodified counterpart (i.e., a corresponding nucleic acid with "normal" backbone, bases, and phosphodiester linkages). A nucleic acid that is acid resistant is preferably at least 1.5 times more resistant to acid degradation, at least 2 times more resistant, even more preferably at least 5 times more resistant, and most preferably at least 10 times more resistant than their unmodified counterpart.

The term "polymer" as used herein refers to a nucleic acid or oligonucleotide molecule having both 1) a modified nucleic acid backbone and 2) at least one end-block at or near the end of the molecule.

The term "LD₅₀" as used herein is the dose of an active substance that will result in 50 per cent lethality in all treated experimental animals. Although this usually refers to invasive administration, such as oral, parenteral, and the like, it may also apply to toxicity using less invasive methods of administration, such as topical applications of the active substance.

The term "alkyl" as used herein refers to a branched or unbranched saturated hydrocarbon chain containing 1-6 carbon atoms, such as methyl, ethyl, propyl, tert-butyl, n-hexyl and the like.

The term "sensitivity" as used herein refers to the relative strength of recognition of a nucleic acid by a binding partner, e.g., an oligonucleotide complementary to the nucleic acids of interest, i.e., PDE4D. The recognition of the binding partner to the sequence of interest must be significantly greater than the recognition of background sequences, and preferably the strength of recognition of the binding partner is at least 10 times, more preferably at least 100 times greater, and even more preferably at least 500 times greater than recognition of background proteins.

The term "selectivity" as used herein refers to the preferential binding of a binding partner to a particular nucleic acid sequence. Preferably, a selective binding partner is at least 10 times, more preferably 100 times, and even more preferably 1000 times more likely to bind to its designated polynucleotide sequence than to any other background sequence.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in a mammal, particularly a human, and includes:
(a) preventing a disease from occurring in a subject which may be predisposed to the disease but has not yet been diagnosed as having it;
(b) inhibiting a disease, i.e., arresting its development; or
(c) relieving a disease, i.e., causing regression of the disease. The invention is generally directed toward treating patients by the administration of a nucleic acid sequence that will modulate expression of an endogenous gene *in vivo.*

By "therapeutically effective" amount is meant a nontoxic but sufficient amount of a compound to provide the desired therapeutic effect, in the present case, that dose of modified nucleic acid which will be effective in relieving, ameliorating, or preventing symptoms of the condition or disease being treated.

### GENERAL ASPECTS OF THE INVENTION

The concept of antisense has been well developed and a number of different antisense therapeutic drugs are in or have completed clinical trials. Antisense therapeutic compounds are oligonucleotides, preferably nuclease resistant, complementary to the mRNA coding for a particular protein. Antisense oligonucleotides generally interfere with the transcription or translation of the targeted gene and thereby reduce expression of the target gene. Because of the great specificity that is possible with antisense there are far fewer, if any, side affects.

The invention also describes either neutral or protonated/acidified oligonucleotide polymers that are substantially nuclease resistant. This example includes oligonucleotide polymers completely or partially derivatized by phosphorothioate linkages, 2'-deoxy-erythropentofuranosyl, 2'-fluoro, 2'-O-alkyl or 2'-O-alkyl-n(O-alkyl) phosphodiesters, p-ethoxy oligonucleotides, p-isopropyl oligonucleotides, phosporamidates, chimeric linkages, and any other backbone modifications. This example also includes other modifications that render the oligonucleotide polymers substantially resistant to endogenous nuclease activity. Additional methods of rendering an oligonucleotide polymer nuclease resistant include, but are not limited to, covalently modifying the purine or pyrimidine bases that comprise the oligonucleotide polymer. For example, bases may be methylated, hydroxymethylated, or otherwise substituted (e.g., glycosylated) such that the oligonucleotide polymers comprising the modified bases are rendered substantially nuclease resistant.

In order to enhance the exonuclease resistance of the oligonucleotide polymers of the invention, the 3' and/or 5' ends of the nucleic acid sequence are preferably attached to an exonuclease blocking function. Also described herein are for example, one or more phosphorothioate nucleotides can be placed at either end of the oligoribonucleotide. Additionally, one or more inverted bases can be placed on either end of the oligonucleotide polymer, or one or more alkyl moieties, e.g., butanol-substituted nucleotides or chemical groups can be placed on one or more ends of the oligonucleotide polymer. 5' and 3' end blocking groups may include one or more phosphorothioate nucleotides (but typically fewer than six), inverted base linkages, or alkyl, alkenyl, or alkynl groups or substituted nucleotides or 2'-O-alkyl-n(O-alkyl). A partial list of blocking groups includes inverted bases, dideoxynucleotides, methylphosphates, alkyl groups, alcohol groups, aryl groups, cordycepin, cytosine arabanoside, 2'-methoxyethoxy nucleotides, phosphoramidates, a peptide linkage, dinitrophenyl group, 2'- or 3'-O-methyl bases with phosphorothioate linkages, 3'-O-methyl bases, fluorescein, cholesterol, biotin, acridine, rhodamine, psoralen, glyceryl, methyl phosphonates, a hexa-ethyloxy-glycol, butanol, hexanol, and 3'-O-alkyls. An enzyme-resistant butanol preferably has the structure OH-CH₂CH₂CH₂CH₂ (4-hydroxybutyl) which is also referred to as a C4 spacer.

Typically, the relative nuclease resistance of a nucleic acid can be measured by comparing the percent digestion of a resistant nucleic acid with the percent digestion of its unmodified counterpart (i.e., a corresponding nucleic acid with "normal" backbone, bases, and phosphodiester linkage). Percent degradation may be determined by using analytical HPLC to assess the loss of full length nucleic acids, or by any other suitable methods (e.g., by visualizing the products on a sequencing gel using staining, autoradiography, fluorescence, etc., or measuring a shift in optical density). Degradation is generally measured as a function of time.

Comparison between unmodified and modified nucleic acids can be made by ratioing the percentage of intact modified nucleic acid to the percentage of intact unmodified nucleic acid. For example, if, after 15 minutes of exposure to a nuclease, 25% (i.e., 75% degraded) of an unmodified nucleic acid is intact, and 50% (i.e., 50% degraded) of a modified nucleic acid is intact, the modified nucleic acid is said to be 2 times (50% divided by 25%) more resistant to nuclease degradation than is the unmodified nucleic acid. Generally, a substantially nuclease resistant nucleic acid will be at least about 1.25 times more resistant to nuclease degradation than an unmodified nucleic acid with a corresponding sequence, typically at least about 1.5 times more resistant, preferably about 2 times more resistant, more preferably at least about 5 times more resistant, and even more preferably at least about 10 times more resistant after 15 minutes of nuclease exposure.

Percent acid degradation may be determined by using analytical HPLC to assess the loss of full length nucleic acids, or by any other suitable methods (e.g., by visualizing the products on a sequencing gel using staining, autoradiography, fluorescence, etc., or measuring a shift in optical density). Degradation is generally measured as a function of time.

Comparison between unmodified and modified nucleic acids can be made by ratioing the percentage of intact modified nucleic acid to the percentage of intact unmodified nucleic acid. For example, if, after 30 minutes of exposure to a low pH environment, 25% (i.e., 75% degraded) of an unmodified nucleic acid is intact, and 50% (i.e., 50% degraded) of a modified nucleic acid is intact, the modified nucleic acid is said to be 2 times (50% divided by 25%) more resistant to nuclease degradation than is the unmodified nucleic acid. Generally, substantially "acid resistant" nucleic acids will be at least about 1.25 times more resistant to acid degradation than an unmodified nucleic acid with a corresponding sequence, typically at least about 1.5 times more resistant, preferably about 1.75 more resistant, more preferably at least 5 times more resistant and even more preferably at least about 10 times more resistant after 30 minutes of exposure at 37°C to a pH of about 1.5 to about 4.5.

Acidification of nucleic acids is the process by which protons (or hydrogen atoms) are added to the reactive sites on a nucleic acid. As the pH is decreased, the number of reactive sites protonated increases and the result is a more highly protonated/acidified nucleic acid. As the pH of the nucleic acid decreases, its bacterial inhibiting activity increases. Accordingly, the nucleic acids of the invention are protonated/acidified to give a pH when dissolved in water of less than pH 7 to about pH 1, or in preferred embodiments, pH 6 to about 1 or pH 5 to about 1. In other preferred embodiments, the dissolved nucleic acids have a pH from pH 4.5 to about 1 or, in a preferred embodiment, a pH of 4.0 to about 1, or, in a more preferred embodiment, a pH of 3.0 to about 1, or, in another preferred embodiment, a pH of 2.0 to about 1.

In another aspect of the present invention the acid resistant polymer is used for treating a patient requiring such treatment comprising administering one or more oligonucleotide polymer(s) targeted to hybridize with one or more of the appropriate PDE4 mRNA(s) in a therapeutically effective amount. In a further aspect, the acid resistant polymer is used for treating a patient requiring such treatment comprising administering one or more oligonucleotide polymer(s) targeted to hybridize with one or more of the PDE4D mRNA(s) in an amount effective to reduce expression of one or more of the PDE4D enzymes. Preferably, the antisense oligonucleotide polymers are in a pharmaceutically acceptable carrier. The targeted PDE4D gene sequence or sequences are selected from the group consisting of the PDE4D genes, their isozymes and their splice variants.

The acid resistant polymers and compositions of the invention are useful as analytical tools in the study of individual PDE4D isoforms and in therapeutic treatment. The acid resistant polymers and compositions of the invention are useful for treating various diseases or disorders, including but not limited to: asthma, hay fever, atopic dermatitis, depression, reperfusion injury, septic shock, toxic shock, endotoxic shock, adult respiratory distress syndrome, autoimmune diabetes, diabetes insipidus, multi-infarct dementia, AIDS, cancer, Crohn's disease, multiple sclerosis, cerebral ischemia, psoriasis, allograft rejection, restenosis, ulcerative colitis, cachexia, cerebral malaria, allergic rhino-conjunctivitis, osteoarthritis, rheumatoid arthritis, chronic bronchitis, eosinophilic granuloma, and autoimmune encephalomyelitis. Particular embodiments of the present invention are directed towards the treatment of the above diseases.

Optionally, the presently described oligonucleotide polymers may be formulated with a variety of physiological carrier molecules. The presently described oligonucleotide polymers may also be complexed with molecules that enhance their ability to enter the target cells. Examples of such molecules include, but are not limited to, carbohydrates, polyamines, amino acids, peptides, lipids, and molecules vital to cell growth. For example, the oligonucleotide polymers may be combined with a lipid, the resulting oligonucleotide polymer/lipid emulsion, or liposomal suspension may, *inter alia,* effectively increase the *in vivo* half-life of the oligonucleotide polymer. The use of cationic, anionic, and/or neutral lipid compositions or liposomes is generally described in International Publications Nos. WO 90/14074, WO 91/16024, WO 91/17424, Pat. No. 4,897,355, herein incorporated by reference.

Alternatively, oligonucleotide polymers directed at PDE4D targets may also be protonated/acidified to function in a dual role as phosphodiesterase inhibitors and antibacterial agents. Accordingly, another embodiment of the presently described invention is the use of a PDE4D modulating therapeutic oligonucleotide polymer that is additionally protonated/acidified to increase cellular uptake, improve encapsulation in liposomes, so it can also serve as an antibiotic.

Additionally, the oligonucleotide polymer may be complexed with a variety of well established compounds or structures that, for instance, further enhance the *in vivo* stability of the oligonucleotide polymer, or otherwise enhance its pharmacological properties (e.g., increase *in vivo* half-life, reduce toxicity, etc.).

### Selection of Antisense Oligonucleotide Polymers

Antisense oligonucleotide polymers may be targeted to particular functional domains of a gene or mRNA transcript. The potential targets include, but are not limited to, regulatory regions, the 5'-untranslated region, the translational start site, the translational termination site, the 3'-untranslated region, exon/intron boundaries and splice sites, as well as sequences internal to the coding region.

As a first step in the selection of a target domain within the target gene, the target gene, pre-mRNA, or mRNA sequence is examined for the presence of homopolymer runs (4 or more bases in a row) within the target sequence, the targeting of which should generally be avoided.

After selection of a target gene, marking the homopolymer runs, and identification of a target domain, a region of the pre-mRNA, mRNA or DNA sequence within that domain is analyzed. An analysis of the segment of the sequence extending approximately 5 to 100 bases or more upstream and downstream from a possible antisense target site is performed. The locations of potentially stable secondary structures are defined as stem-loop structures with predicted melting temperatures above 20°C. This analysis can be performed using commercially available software such as OLIGO 4.0 for Mac, or OligoTech. Sequences involved in stem hybridization for loop-stem secondary structure formation are regarded as part of the secondary structure and are treated as a structural unit for the purposes of analysis and selection of an antisense oligonucleotide polymer. In one embodiment of this target selection technique, antisense oligonucleotide polymers can be targeted to sequences of the gene, pre-mRNA, or mRNA with predicted secondary structure melting temperatures of less than 100°C (according to the commercially available analysis programs). In a preferred embodiment, the secondary structure melting temperatures would be less than 60°C. In a further preferred embodiment, the secondary structure melting temperature would be less than 40°C. In a most preferred embodiment, secondary structure melting temperatures would be less than 20°C.

Oligonucleotide polymer sequences with stable loop structures or which form stable dimers should be avoided. In one embodiment of this target selection technique, antisense oligonucleotide polymers with predicted secondary structure melting temperatures of less than 100°C (according to the commercially available analysis programs) are chosen. In a preferred embodiment the secondary structure melting temperatures would be less than 80°C. In a more preferred embodiment the secondary structure melting temperatures would be less than 60°C. In a further preferred embodiment the secondary structure melting temperatures would be less than 40°C. In the most preferred embodiment, the secondary structure melting temperature is less than 20°C. The formation of stable secondary or tertiary structure by the antisense oligonucleotide polymers may potentially compete with their ability to bind to the target DNA or RNA. Similarly, formation of homodimers by the antisense oligonucleotide polymers may compete with their ability to bind to target DNA or RNA. Homopolymer runs of a single base ideally will be three bases or less within the oligonucleotide polymer sequence. Generally, within a target, those sequences with the lowest secondary structure melting temperatures (loop Tm), or no secondary structure work best.

Sequences of antisense oligonucleotide polymers useful as compositions and in methods of the present invention include the following:
Target Gene: PDE4A (PDE4A) - - Acc# U68532 (SEQ ID NO:46), which is not part of the present invention

| Target Domain (Nucleotide Position #s) | Antisense Oligonucleotide Sequence |
|---|---|
| 59-35 | tta gag cag gtc tcg cag aag aaa t, (SEQ ID NO: 1) |
| 569-545 | agc gtc agc atg tat gtc acc atc g, (SEQ ID NO: 2) |
| 886-862 | gct tgc tga ggt tct gga aga tgt c, (SEQ ID NO: 3) |
| 1541-1518 | aga gct tcc tcg act cct gac aat, (SEQ ID NO: 4) |
| 359-335 | atg tta gag ttg ttc agg ctg tta c, (SEQ ID NO: 5) |
| 398-374 | agg agc tct tct tga tcg gtc ttc a, (SEQ ID NO: 6) |
| 696-674 | gag aat ctc cag gtc cgt gaa ca, (SEQ ID NO: 7) |
| 1350-1333 | ggc gct gta gta cca gtc, (SEQ ID NO: 8) |
| 2797-2782 | aca ggg aca gag gtc t, (SEQ ID NO: 9) |
| 3445-3421 | gac ttc tag tca gta tcg cca gga g, (SEQ ID NO: 10) |

Target Gene: PDE4B (PDE4B2B) -- Acc# L20971 (SEQ ID NO:47), which is not part of the present invention

| Target Domain (Nucleotide Position #s) | Antisense Oligonucleotide Sequence |
|---|---|
| 720-696 | aca aat cac agt ggt gct ctg cct g, (SEQ ID NO: 11) |
| 1441-1417 | ggt ctt cta aag tca tca tgt agg t. (SEQ ID NO: 12) |
| 2185-2166 | cta agg tat cga gaa tgt cc, (SEQ ID NO: 13) |
| 2506-2482 | cat gct cat caa gga tag aat gtt c, (SEQ ID NO: 14) |
| 269-247 | gac gtt tgg gtt ata taa tac ac, (SEQ ID NO: 15) |
| 995-971 | ttg tta gaa gcc atc tca ctg aca g, (SEQ ID NO: 16) |
| 1787-1763 | atg tca ata acc atc ttc ctg agt g, (SEQ ID NO: 17) |
| 1889-1865 | tct agg aga aga agc cct gaa ctt g, (SEQ ID NO: 18) |
| 3254-3235 | gag ttc tat gca gac tct ca, (SEQ ID NO: 19) |
| 3951-3927 | tca gta gtt cgg gag cat tca gaa g, (SEQ ID NO: 20) |

Target Gene: PDE4C (PDE4C1B) -- Acc# Z46632 (SEQ ID NO:48), which is not part of the present invention

| Target Domain (Nucleotide Position #s) | Antisense Oligonucleotide Sequence |
|---|---|
| 119-102 | ttc tcc atg cgc cag aga, (SEQ ID NO: 21) |
| 532-515 | cag agg agt tcc gag aca, (SEQ ID NO: 22) |
| 1681-1664 | cga ggc ttg tca cct tct, (SEQ ID NO: 23) |
| 2255-2231 | tgg ccc taa gtc ctc tgg ttg tcg a, (SEQ ID NO: 24) |
| 69-52 | gct tgg ctg ctc cta ggc, (SEQ ID NO: 25) |
| 560-538 | atg tcc tct cca tgt agg tcg ct, (SEQ ID NO: 26) |
| 1140-1121 | gtt ccc act tac gtc cgc ca, (SEQ ID NO: 27) |
| 1360-1336 | cca agt ctg tga aca cag cct cga g, (SEQ ID NO: 28) |
| 2003-1979 | cga ttg tcc tcc agc gtg tcc age a, (SEQ ID NO: 29) |
| 3095-3071 | agt cat agc tct ctt cag cct cca a, (SEQ ID NO: 30) |

Target Gene: PDE4D (PDE4D2A) -- Acc# U50158 (SEQ ID NO:49)

| Target Domain (Nucleotide Position #s) | Antisense Oligonucleotide Sequence |
|---|---|
| 1683-1659 | ttg cac tgt tac gtg tca gga gaa c, (SEQ ID NO: 31) |
| 1672-1659 | cgt gtc agg aga ac, (SEQ ID NO: 32) |
| 1645-1621 | aca ggc ttc agg ttg gct ttc ctc t, (SEQ ID NO: 33) |
| 1127-1103 | gag gct ttg ttg ggt tgc tca gat c, (SEQ ID NO: 34) |
| 682-658 | aga taa tag cac atg agt aga ctg g, (SEQ ID NO: 35) |
| 154-130 | cat ctc act gac gga gtg cct ggt c, (SEQ ID NO: 36) |
| 609-592 | taa tgg tct tcg aga gtc, (SEQ ID NO: 37) |
| 822-798 | ttg tac atc aag gca agt tca gag t, (SEQ ID NO: 38) |
| 1046-1022 | gaa gaa ctc cag age ttg tca ctt t, (SEQ ID NO: 39) |
| 791-767 | tga tca gaa att gat tgg aca cac c, (SEQ ID NO: 40) |
| 1371-1347 | tgg tac cat tca cga ttg tcc tcc a, (SEQ ID NO: 41) |

Target Gene: PDE4D (PDE4D5A)-- Acc# AF012073 (SEQ ID NO:50)

| Target Domain (Nucleotide Position #s) | Antisense Oligonucleotide Sequence |
|---|---|
| 300-276 | tct cgg aga gat cac tgg aga gag c, (SEQ ID NO: 42) |
| 375-351 | atg tgc cac cgt gaa acg ccg ctg t, (SEQ ID NO: 43) |

Target Gene: PDE4D (PDE4D3) -- Acc# L20970 (SEQ ID NO:51)

| Target Domain (Nucleotide Position #s) | Antisense Oligonucleotide Sequence |
|---|---|
| 95-76 | tcc tac gtt aca tgt agt ga, (SEQ ID NO: 44) |
| 178-159 | cat atc cag gaa tgc ctt ct, (SEQ ID NO: 45) |

### In Vivo Testing of Oligonucleotide Polymers

When used in the therapeutic treatment of disease, an appropriate dosage of an antisense PDE4D oligonucleotide polymer, or mixture thereof, may be determined by any of several well established methodologies. For instance, animal studies are commonly used to determine the maximal tolerable dose, or MTD, of bio-active agent per kilogram weight. In general, at least one of the animal species tested is mammalian. Those skilled in the art regularly extrapolate doses for efficacy and avoiding toxicity to other species, including human. Additionally, therapeutic dosages may also be altered depending upon factors such as the severity of infection and the size or species of the host.

Oligonucleotide polymers are preferably administered in a pharmaceutically acceptable carrier, via oral, intranasal, rectal, topical, intraperitoneal, intramuscular, subcutaneous, intracranial, subdermal, transdermal, intratracheal methods, or the like.

For example, topical diseases are preferably treated or prevented by formulations designed for topical application. Alternately, where the targeted disease state is in the stomach or gut, preparations of oligonucleotide polymers may be provided by oral dosing. Additionally, pulmonary sites of disease, e.g., asthma, may be treated both parenterally and by direct application of suitably formulated forms of the oligonucleotide polymers to the lung by inhalation therapy.

Where suitably formulated oligonucleotide polymers are administered parenterally, the oligonucleotide polymers can accumulate to relatively high levels in the kidneys, liver, spleen, lymph glands, adrenal gland, aorta, pancreas, bone marrow, heart, and salivary glands. Oligonucleotide polymers also tend to accumulate to a lesser extent in skeletal muscle, bladder, stomach, esophagus, duodenum, fat, and trachea. Still lower concentrations are typically found in the cerebral cortex, brain stem, cerebellum, spinal cord, cartilage, skin, thyroid, and prostate (see generally Crooke, 1993, Antisenve Research and Applications, CRC Press, Boca Raton, FL).

One of ordinary skill will appreciate that, from a medical practitioner's or patient's perspective, virtually any alleviation or prevention of an undesirable symptom would be desirable. Thus, the terms "treatment", "therapeutic use", or "medicinal use" used herein shall refer to any and all uses of the claimed oligonucleotide polymers that remedy a disease state or symptoms, or otherwise prevent, hinder, retard, or reverse the progression of disease or other undesirable symptoms in any way whatsoever.

Preferably, animal hosts that may be treated using the oligonucleotide polymers of the present invention include, but are not limited to, invertebrates, vertebrates, birds, mammals such as pigs, goats, sheep, cows, dogs, cats, and particularly humans. Oligonucleotide polymers are designed to be appropriate to the particular animal to be treated.

Another embodiment of the presently described invention is the use of a therapeutic oligonucleotide polymer that is additionally protonated/acidified so it can also serve as an antibiotic. Purified or crude nucleic acids can be protonated/acidified with acid, including but not limited to, phosphoric acid, nitric acid, hydrochloric acid, acetic acid, etc. For example, acid may be combined with nucleic acids in solution, or alternatively, the nucleic acids may be dissolved in an acidic solution. Excess acid may be removed by chromatography or in some cases by drying the nucleic acid.

Other procedures to prepare protonated nucleic acids known to the skilled artisan are equally contemplated to be within the scope of the invention. Once the nucleic acids of the present invention have been protonated they may be separated from any undesired components such as excess acid. The skilled artisan would know of many ways to separate the oligonucleotide polymers from undesired components. For example, the oligonucleotide polymer solution may be subjected to chromatography following protonation. In a preferred embodiment, the oligonucleotide polymer solution is run over a poly(styrene-divinylbenzene) based resin column (e.g., Hamilton's PRP or Polymer Labs' PLRP) following protonation.

The protonated/acidified nucleic acids can be used directly, or in a preferred embodiment, processed further to remove any excess acid and salt via precipitation, reverse phase chromatography, diafiltration, or gel filtration. The protonated/acidified oligos can be concentrated by precipitation, lyophilization, solvent evaporation, etc. When suspended in water or saline, the acidified nucleic acid preparations of the invention typically exhibit a pH between 0.5 and 4.5 depending upon 1) the level of protonation/acidification, which can be determined by how much acid is used in the acidification process, and 2) the concentration of the nucleic acid. Alternatively, nucleic acids can be protonated by passage over a cation exchange column charged with hydrogen ions.

It has been discovered that protonated oligonucleotide polymers, in a sequence independent manner, exhibit antibacterial properties. The protonated oligonucleotide polymers of the invention are protonated/acidified to give a pH when dissolved in water of less than pH 7 to about pH 1, or in preferred embodiments, pH 6 to about 1 or pH 5 to about 1. In other preferred embodiments, the dissolved nucleic acids have a pH from pH 4.5 to about 1 or, in a preferred embodiment, a pH of 4.0 to about 1, or, in a more preferred embodiment, a pH of 3.0 to about 1, or, in another preferred embodiment, a pH of 2.0 to about 1.

### Pharmaceutical Compositions and Delivery

Oligonucleotide polymers may be formulated with a variety of physiological carrier molecules for *in vivo* use. For example, the oligonucleotide polymers may be combined with a lipid, cationic lipid, or anionic lipid (which may be preferred for protonated/acidified oligonucleotide polymers) and the resulting oligonucleotide polymer/lipid emulsion, or liposomal suspension may, *inter alia,* effectively increase the *in vivo* half-life of the oligonucleotide polymer.

When used in the therapeutic treatment of disease, an appropriate dosage of a PDE4 modulating oligonucleotide polymer, or mixture thereof, may be determined by any of several well established methodologies. For instance, animal studies are commonly used to determine the maximal tolerable dose, or MTD, of bio-active agent per kilogram weight. In general, at least one of the animal species tested is mammalian. Those skilled in the art regularly extrapolate doses for efficacy and avoiding toxicity to other species, including human. Additionally, therapeutic dosages may also be altered depending upon factors such as the severity of the disease, and the size or species of the host.

The use of protonated/acidified oligonucleotide polymers facilitates their encapsulation in anionic lipids which are thought to have a number of advantages over cationic liposomes (R.J. Lee and L. Huang, "Lipidic Vector Systems for Gene Transfer", in Critical Reviews in Therapeutic Drug Carrier Systems 14(2):173-206 (1997)). Specifically, anionic liposomes are likely to be less toxic than cationic liposomes, they exhibit lower non-specific uptake, and they can be targeted with the appropriate ligands to specific cells.

Examples of suitable anionic lipids for use with protonated/acidified oligonucleotide polymers include, but are not limited to, cardiolipin, dimyristoyl, dipalmitoyl, or dioleoyl phosphatidyl choline or phosphatidyl glycerol, palmitoyloleoyl phosphatidyl choline or phosphatidyl glycerol, phosphatidic acid, lysophosphatidic acid, phosphatidyl serine, phosphatidyl inositol, and anionic forms of cholesterol. The use of cationic, anionic, and/or neutral lipid compositions or liposomes is generally described in International Publications Nos. WO 90/14074, WO 91/16024, WO 91/17424, and U.S. Pat. No. 4,897,355,

By assembling oligonucleotide polymers into lipid-associated structures, the oligonucleotide polymers may be targeted to specific cell types by the incorporation of suitable targeting agents (i.e., specific antibodies or receptors) into the oligonucleotide polymer/lipid complex.

Pharmaceutical compositions containing oligonucleotide polymers of the invention in intimate admixture with a pharmaceutical carrier can be prepared according to conventional pharmaceutical compounding techniques. The carrier may take a wide variety of forms depending on the form of preparation desired for administration, e.g., intravenous, oral, topical, aerosol (for topical or inhalation therapy), suppository, parenteral, or spinal injection.

In preparing the compositions in oral dosage form, any of the usual pharmaceutical media may be employed, such as, for example, water, glycols, oils, alcohols, flavoring agents, preservatives, coloring agents and the like in the case of oral liquid preparations (such as, for example, suspension, elixirs, and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques.

For parenteral application by injection, preparations may comprise an aqueous solution of a water soluble, or solubilized, and pharmaceutically acceptable form of the oligonucleotide polymer in an appropriate saline solution. Injectable suspensions may also be prepared using appropriate liquid carriers, suspending agents, agents for adjusting the isotonicity, preserving agents, and the like. Actual methods for preparing parenterally administrable compositions and adjustments necessary for administration to subjects will be shown or apparent to those skilled in the art and are described in more detail in, for example, Remington's Pharmaceutical Science, 15th Ed., Mack Publishing Company, Easton, PA (1980), which is incorporated herein by reference. The presently described oligonucleotide polymers should be parenterally administered at concentrations below the maximal tolerable dose (MTD) established for the oligonucleotides.

For topical administration, the carrier may take a wide variety of forms depending on the preparation, which may be a cream, dressing, gel, lotion, ointment, or liquid.

Aerosols are prepared by dissolving or suspending the oligonucleotide in a propellant such as ethyl alcohol or in propellant and solvent phases. The pharmaceutical compositions for topical or aerosol form will generally contain from about 0.01% by weight (of the oligonucleotide) to about 40% by weight, preferably about (0.02% to about 10% by weight, and more preferably about 0.05% to about 5% by weight depending on the particular form employed.

Suppositories are prepared by mixing the oligonucleotide polymer with a lipid vehicle such as theobroma oil, cacao butter, glycerin, gelatin, or polyoxyethylene glycols.

The presently described oligonucleotide polymers may be administered to the body by virtually any means used to administer conventional therapeutics. A variety of delivery systems are well known in the art for delivering bioactive compounds to an animal. These systems include, but are not limited to, intravenous or intra-muscular or intra-tracheal injection, nasal spray, aerosols for inhalation, and oral or suppository administration. The specific delivery system used depends on the location of the disease, and it is well within the skill of one in the art to determine the location of the disease and to select an appropriate delivery system.

### EXAMPLES

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g., amounts, temperature, concentrations, etc.) but some experimental errors and deviations should be allowed for. Unless otherwise indicated, parts are parts by weight, molecular weight is average molecular weight, temperature is in degrees centigrade, and pressure is at or near ambient.

### EXAMPLE 1: B Cell Mediated Dermatitis

An anti-inflammatory oligonucleotide polymer, OE-2a (SEQ ID NO: 32), is a 2' O-methyl RNA phosphodiester linked, with 5' and 3' ends blocked with inverted Ts, and is targeted against the human PDE4 gene. OE-2a was dissolved at 35 mg/ml (7.7 µMolar) in water at approximately pH 3 and used to treat a 37 year old male with a history of severe recurrent atopic dermatitis. The dermatitis was specifically eradicated with OE-2a. In addition, future occurrences of atopic dermatitis have been completely eliminated.

A 37 year old male presented with severe atopic dermatitis, completely covering the inside of both forearms. Atopic dermatitis is a chronic disorder characterized by intensely pruritic inflamed papules. The inflammatory response is associated with over-production of IgE by B lymphocytes. Higher levels of PDE4 have been reported for individuals with atopic dermatitis. An antisense oligonucleotide polymer, OE-2a, specifically targeted to PDE4 was applied to a 2" by 3" segment of the left forearm. A second oligo, OE-1, was applied to another segment of the same arm. OE-1 has a similar base distribution to OE-2a, but is homologous to a bacterial gene. Oligonucleotide polymers were applied twice at 12 hour intervals.

OE-2a was successful at clearing the dermatitis on the area to which it was applied. OE-1 had no effect. The patient commented prior to the second treatment that the itching had stopped on the area corresponding to the OE-2a after about 6 hours.

Treatment on the second arm was initiated at this time exclusively with OE-2a. Again there were 2 treatments 12 hours apart. The patient remarked that all itching had ceased within 6 hours. The dermatitis was completely cleared within 12 hours of the second treatment.

There have been no recurrences of atopic dermatitis on this individual from February through December of a single year, although he typically suffers from recurring bouts during the heat of the summer.

### EXAMPLE 2: T Cell Mediated Dermatitis

OE-2a at 35 mg/ml (7.7 µMolar) in water at approximately pH 3 was used to treat a case of T cell mediated contact dermatitis triggered by poison ivy. The dermatitis was completely eliminated with 2 treatments, and secondary eruptions on other areas of the infected individual were prevented.

A female presented with poison ivy on several areas of the bottom of her foot. The patient complained of itching that is associated with T cell mediated type hypersensitivity. The contact dermatitis was characterized by redness, induration and vesiculation. The anti-inflammatory oligo OE-2a was applied to the bottom of the foot. Within 12 hours the patient noted that the intense itching had ceased. A second application of OE-2a resulted in a disappearance of the redness, induration, and vesiculation. It is of interest to note that topical application of OE-2a not only eliminated the visible poison ivy on the bottom of the foot, but also prevented any additional eruptions of poison ivy induced dermatitis elsewhere on the individual.

### EXAMPLE 3: Acute Wheal and Flare Reaction

OE-2a at 43.6 mg/ml (7.7 µMolar) and approximately pH 7 was able to prevent both an immediate and delayed-type hypersensitivity response when applied within minutes of receiving multiple wasp stings.

A female patient with a history of severe wheal and flare skin reaction in response to insect bites presented with an arm that had received multiple wasp stings. The patient was in severe pain and hysterical. OE-2a was administered immediately. Within 5 minutes the patient was calm and pain free. Remarkably, administration of the OE-2a within 5 minutes of receiving the stings completely prevented any immediate wheal and flare reaction, as well as any delayed reaction.

### EXAMPLE 4: Chemically Induced Dermatitis

OE-2a was successfully used to treat a case of chemically induced contact dermatitis that had failed to respond to standard dermatological treatments.

A 59 year old woman presented with a case of contact dermatitis on her neck triggered by a reaction to dry cleaning reagents in a turtle neck sweater. Over the course of a month she had been treated by a dermatologist with cortisone, antibiotics, and a variety of other topical drugs. The various treatments gave no more than temporary sporadic relief and they failed to eliminate the itching and redness. At this time the patient was treated topically with OE-2a. The itching and redness disappeared completely after two treatments with no recurrences over a 5 month period.

### EXAMPLE 5: Intranasal Administration of PDE-4

Several healthy male volunteers with severe allergic rhinitis were treated with OE-2a. The males ranged in age from 45 years old to 73 years old. The weights ranged from 175 pounds to 230 pounds. A sterile nasal spray container was filled with a solution containing OE-2a dissolved in normal saline. The solution of oligonucleotide polymer in saline, at a concentration of concentration of approximately 300 A₂₆₀ per ml, was administered via the intranasal route twice daily to each nostril. This dosage regime was continued for three days.

Upon examination, following the three day course of treatment, all volunteers were found to be completely free of all symptoms of allergic rhinitis. Specifically there was no nasal congestion, no nasal discharge, no sneezing, no difficulty breathing through the nose, and the nasal passages appeared to be completely clear. There has been no relapse of any of the symptoms of rhinitis since treatment schedule was completed, although the volunteers have continued to be exposed to the allergens that triggered the rhinitis. In conclusion, intranasal administration of PDE-4 dissolved in saline and administered via an intranasal spray appeared to cure allergic rhinitis in healthy male volunteers after only three days of treatment, with no further recurrences of the symptoms.

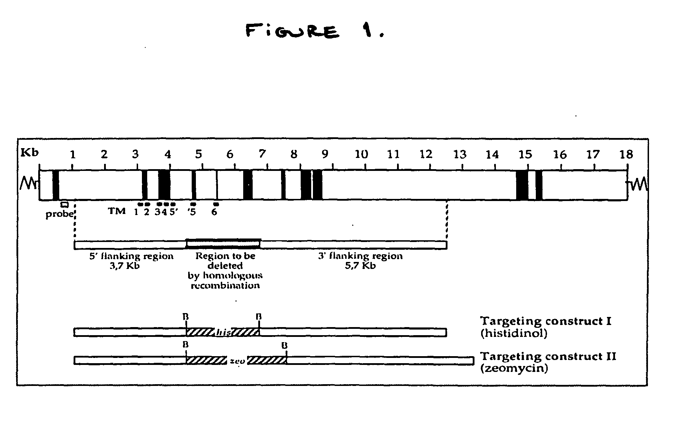

## Claims

1. An acid resistant polymer complementary to PDE4D for modulating PDE4D activity comprising:
- a nucleic acid having a backbone structure modified from that of a naturally occurring nucleotide polymer; and
- a blocking chemical modification at or near at least one end of the nucleic acid;
wherein the polymer is **characterized by** a pH stability of at least 30 minutes at a pH of about 1.5 to about 4.5 and a nuclease resistance of at least twice that of a naturally occurring nucleic acids having the same number of nucleotides, wherein the nucleic acid has a sequence selected from the group consisting of: SEQ ID NO: 31; SEQ ID NO: 32; SEQ ID NO: 33; SEQ ID NO: 34; SEQ ID NO: 35; SEQ ID NO: 36; SEQ ID NO:37; SEQ ID NO: 38; SEQ ID NO: 39; SEQ ID NO: 40; SEQ ID NO: 41; SEQ ID NO: 42; SEQ ID NO: 43; SEQ ID NO: 44; and SEQ ID NO: 45.

2. The polymer of claim 1, wherein the backbone structure includes one or more of the group consisting of: 2'-O-methyl, 2'-O-alkyl and 2'-O-alkyl-n(O-alkyl).

3. The polymer of claims 1 or 2, wherein the polymer hybridizes to PDE4D DNA sequences.

4. The polymer of claims 1 or 2, wherein the polymer hybridizes to a PDE4D mRNA.

5. The polymer of claims 1, 2, 3 or 4, wherein the polymer is protonated to have a pH below 7.

6. A pharmaceutical composition comprised of a polymer of any of claims 1-5; and a pharmaceutically acceptable carrier.

7. The pharmaceutical composition of claim 6, wherein said polymer is protonated to have a pH below 7.

8. One or more of the acid resistant polymers according to any one of claim 1-5 for use as a medicament.

## Patentansprüche

1. Ein zu PDE4D komplementäres, säureresistentes Polymer zur Modulation der PDE4D Aktivität, das umfasst:
- eine Nukleinsäure mit einer Rückgratstruktur, die in Bezug auf ein natürlich auftretendes Nukleotidpolymer modifiziert ist;
- eine chemische Modifikation an oder in der Nähe mindestens eines Endes der Nukleinsäure zum Blockieren;
wobei das Polymer durch eine pH-Stabilität von mindestens 30 Minuten bei einem pH von ungefähr 1.5 bis ungefähr 4.5 und einer Nukleasebeständigkeit von mindestens zweimal der natürlich auftretender Nukleinsäuren mit der gleichen Anzahl von Nukleotiden charakterisiert ist, wobei die Nukleinsäure eine Sequenz hat, die aus der Gruppe, die aus SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; und SEQ ID NO: 45 besteht, ausgewählt wird.

2. Das Polymer nach Anspruch 1, wobei die Rückgratstruktur eines oder mehrere aus der Gruppe, die aus 2'-O-Methyl, 2'-O-Alkyl und 2'-O-Alkyl-n(O-alkyl) besteht, beinhaltet.

3. Das Polymer nach den Ansprüchen 1 oder 2, wobei das Polymer an PDE4D DNA Sequenzen hybridisiert.

4. Das Polymer nach den Ansprüchen 1 oder 2, wobei das Polymer an eine PDE4D mRNA hybridisiert.

5. Das Polymer nach den Ansprüchen 1, 2, 3 oder 4, wobei das Polymer protoniert ist, um einen pH unterhalb 7 zu haben.

6. Eine pharmazeutische Zusammensetzung, die ein Polymer nach einem der Ansprüche 1-5 und einen pharmazeutisch geeigneten Träger umfasst.

7. Die pharmazeutische Zusammensetzung nach Anspruch 6, wobei das Polymer protoniert ist, um einen pH unterhalb von 7 zu haben.

8. Ein oder mehrere säureresistente Polymere gemäß einem der Ansprüche 1-5 für die Verwendung als ein Medikament.

## Revendications

1. Polymère résistant aux acides complémentaire de PDE4D pour moduler l'activité PDE4D comprenant :
- un acide nucléique ayant une structure squelettique modifiée par rapport à celle d'un polymère nucléotidique naturel ; et
- une modification chimique bloquante au niveau ou proche d'au moins une extrémité de l'acide nucléique ;
dans lequel le polymère est **caractérisé par** une stabilité au pH d'au moins 30 minutes à un pH d'environ 1,5 à 4,5 et une résistance aux nucléases d'au moins deux fois celle d'un acide nucléique naturel ayant le même nombre de nucléotides, dans lequel l'acide nucléique a une séquence choisie dans le groupe consistant en: SEQ ID NO: 31 ; SEQ ID NO: 32 ; SEQ ID NO: 33 ; SEQ ID NO: 34 ; SEQ ID NO: 35 ; SEQ ID NO: 36 ; SEQ ID NO: 37 ; SEQ ID NO: 38 ; SEQ ID NO: 39 ; SEQ ID NO: 40 ; SEQ ID NO: 41 ; SEQ ID NO: 42 ; SEQ ID NO: 43 ; SEQ ID NO: 44 ; et SEQ ID NO: 45.

2. Polymère selon la revendication 1, dans lequel la structure squelettique comprend un ou plusieurs groupements du groupe consistant en : 2'-O-méthyle, 2'-O-alkyle et 2'-O-alkyl-n(O-alkyl).

3. Polymère selon les revendications 1 ou 2, dans lequel le polymère s'hybride aux séquences d'ADN de PDE4D.

4. Polymère selon les revendications 1 ou 2, dans lequel le polymère s'hybride à un ARNm de PDE4D.

5. Polymère selon l'une des revendications 1, 2, 3 ou 4, dans lequel le polymère est protoné pour avoir un pH inférieur à 7.

6. Composition pharmaceutique composée d'un polymère selon l'une quelconque des revendications 1 à 5, et d'un vecteur pharmaceutiquement acceptable.

7. Composition pharmaceutique selon la revendication 6, dans laquelle ledit polymère est protoné pour avoir un pH inférieur à 7.

8. Utilisation comme médicament d'un ou plusieurs des polymères résistants aux acides selon l'une quelconque des revendications 1 à 5.
